(19) Europäisches Patentamt
European Patent Office
Office européen des brevets

(11) Publication number: **0 021 644 B1**

(12) # EUROPEAN PATENT SPECIFICATION

(45) Date of publication of patent specification: **03.09.86**

(51) Int. Cl.[4]: **C 07 D 333/24**

(21) Application number: **80301854.8**

(22) Date of filing: **04.06.80**

(54) **A salt of 3-thienylmalonic acid and a process for the preparation of 3-thienylmalonic acid.**

(30) Priority: **19.06.79 US 49875**

(43) Date of publication of application:
**07.01.81 Bulletin 81/01**

(45) Publication of the grant of the patent:
**03.09.86 Bulletin 86/36**

(84) Designated Contracting States:
**AT BE CH DE FR GB IT LI LU NL SE**

(56) References cited:
**EP-A-0 021 645**
**GB-A-1 139 164**
**US-A-2 513 140**

(73) Proprietor: **BEECHAM GROUP PLC**
**Beecham House Great West Road**
**Brentford Middlesex TW8 9BD (GB)**

(72) Inventor: **Malik, Shaukat Hussain**
**76 Hidden Lake Drive**
**North Brunswick New Jersey 08902 (US)**
Inventor: **Windus, Charles Edward**
**225 Grove Street**
**Sommerville New Jersey 08876 (US)**
Inventor: **Clark, Dennis Edward**
**239D Blossom Drive RD No 1**
**Basking Ridge New Jersey 07920 (US)**

(74) Representative: **Hesketh, Alan, Dr. et al**
**European Patent Attorney**
**Beecham Pharmaceuticals**
**Great Burgh Tew Tree Bottom Road**
**Epsom, Surrey, KT18 5XQ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 021 644 B1

**Description**

This invention relates to a chemical process for the production of 3-thienylmalonic acid, a compound useful as an intermediate in the production of penicillins of general formula (I):

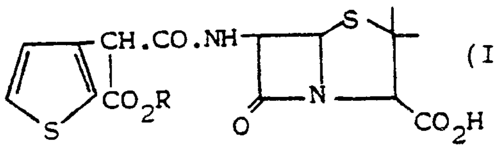

(I)

The compound of formula (I) in which R is hydrogen is disclosed in our British Patent No. 1,004,670, and the sodium salt thereof is known as ticarcillin. Compounds of formula (I) in which R is an alkyl, aralkyl or ring substituted aralkyl group are disclosed in our British Patent No. 1,125,557 and compounds of formula (I) in which R is aryl are disclosed in our British Patent No. 1,133,886.

In British Patent No. 1,125,557 the penicillin of formula (I) was prepared from a 3-thienylmalonic ester itself synthesised from 3-thienylacetonitrile via 3-thienylacetic acid and a diester of 3-thienylmalonic acid. US—A—2 513 140 discloses the compound 3-thienyl acetic acid and a process for its preparation by hydrolysis of 3-thienyl-acetonitrile. Also, GB—A—2009 158, GB—A—1359 991 and GB—A—1359 992 describe prior art processes for preparing 3-thienylmalonic acid. It has now been found that 3-thienylmalonic acid can be prepared in good yield in a one-step process directly from a 3-thienyl cyanoacetate, via the intermediate precipitation of an alkali metal or ammonium salt to 3-thienylmalonic acid.

Accordingly the present invention provides a salt of 3-thienylmalonic acid of formula (II):

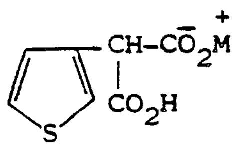

(II)

wherein $\overset{+}{M}$ represents an alkali metal ion or an ammonium ion, $\overset{+}{N}H_4$.

Suitable alkali metal ions include sodium, potassium and lithium. A prefered compound of formula (II) is the monosodium salt of 3-thienylmalonic acid, which appears to exist in the form of the monohydrate.

The invention also provides a process for the preparation of 3-thienylmalonic acid of formula (III):

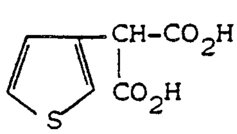

(III)

which process compirses hydrolying a 3-thienylcyanoacetate of formula (IV):

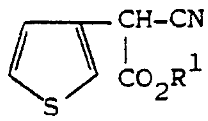

(IV)

wherein $R^1$ represents an alkyl or aryl group, with an alkali metal hydroxide, isolating a compound of formula (II) above and converting it to 3-thienylmalonic acid.

In compound (IV), the group $R^1$ may suitably be $C_{1-6}$ alkyl, phenyl or substituted phenyl. The group $R^1$ is preferably $C_{1-6}$ alkyl, for example methyl or ethyl, especially ethyl.

The compound (IV) may be prepared by conventional methods, for example by treatment of 3-thienylacetonitrile with a compound of formula $R^1O.CO.OR^1$ in the presence of a strong base such as an alkali metal alkoxide, particularly sodium methoxide. Alternatively anhydrides of formula $R^1.CO.O.CO.R^1$ or chloroformic esters of formula $C1.CO_2R^1$ may be used in the presence of an alkali metal alkoxide.

The process of the present invention employs an alkali metal hydroxide. Suitable such hydroxides include sodium hydroxide and potassium hydroxide. The most suitable solvent for this hydrolysis reaction is water aithough it is possible to add a co-solvent. The reaction is preferably carried out at a temperature greater than 40°C. In general if the temperature is maintained at 75° to 80°C the hydrolysis is complete in about 2—2.5 hours. At a higher temperature, for example 100° to 110°C, the time to the hydrolysis is reduced to about 30 to 40 minutes.

It is preferred that a means is provided for removing ammonia vapour which is evolved during the reaction of the invention. This may be achieved for example by blowing an inert gas such as nitrogen through the reaction mixture or by evacuating the space above the reaction mixture. If no such means is provided for removing the ammonia, the process proceeds more slowly.

After the hydrolysis reaction is complete the compound of the formula (II) may be isolated by washing with a water-immiscible solvent such as methyl iso-butyl ketone, conveniently at pH 5.5 to 6 to remove by-products from the reaction, then acidifying the reaction mixture, for example with hydrochloric acid, and extracting the resulting aqueous solution containing 3-thienylmalonic acid into a water-immiscible solvent, such as methyl isobutyl ketone. The salt of formula (II) may be precipitated from such a solution by adding an alkali metal or ammonium salt of an alkanoic acid. A preferred such salt is an alkali metal or ammonium ethyl hexoate, in particular sodium ethyl hexoate, and it is suitably added in solution in the same solvent as that containing the crude 3-thienylmalonic acid.

The salt of 3-thienyl malonate which is thus precipitated need not be further purified. Its precipitation alone is sufficient to remove impurities from the reaction mixture which would otherwise impede the isolation of 3-thienylmalonic acid.

The isolated salt of formula (II) may conveniently be converted into the free acid, 3-thienylmalonic acid, by conventional methods, for example by treatment of an aqueous solution thereof with hydrochloric acid. It is often preferable to seed the solution with a crystal of 3-thienylmalonic acid in order to encourage crystallisation.

The conversion of 3-thienylmalonic acid to a penicillin of formula (I) may be carried out by any convenient method. In particular, those methods described in British Patent specification Nos. 1,004,670, 1,125,557, 1,133,886 and 1,197,973 may be used to advantage.

An important advantage of the process of this invention is that the starting materials may be employed in crude form. For example the 3-thienylcyanoacetate of formula (IV) may be used in 70% or less purity. If 3-thienylmalonic acid is prepared from such impure starting materials by direct precipitation from aqueous or organic solvents, without the intermediate isolation of the salts of formula (II) of this invention, the resulting 3-thienylmalonic is coloured and impure. The present process therefore has considerably commercial advantages in that 3-thienylmalonic acid can be prepared in pure form using starting material in unpurified crude form.

The following Examples illustrate the present invention.

Example 1

Preparation of monosodium 3-thienylmalonate

Charge a 2 litre, round-bottom flask with 208 g sodium hydroxide and water (650 ml), and heat to 105°C. Add ethyl-3-thienylcyanoacetate (130.5 g) over 20—30 minutes, maintaining 100°—105°C, distilling out low boiling material. Stir 10 minutes, cool to 15°—20°C, add 30 ml concentrated HCl, 500 ml methyl isobutyl ketone (MIBK), and approximately 65 ml more concentrated HCl to pH 5.8—6.0. Stir 15 minutes, filter through filter aid, wash cake with 100 ml water and separate the layers. To the aqueous layer add 500 ml MIBK, sitr 15 minutes at 20°—25°C, pH 5.5—6.0 (adjust with ca. 2 ml concentrated HCl). Separate the layers. Wash the MIBK layer with 30 ml water, and separate the layers again. Treat the combined aqueous layers with 20 g charcoal (Nuchar SN) for 0.5 hours at 20°C—25°C, filter through a filter aid, wash the cake with 4 × 60 ml water. To the filtrate add 150 g sodium chloride, stir to dissolve, add 350 ml MIBK, and at 20°—25°C add concentrated HCl to pH 1.0—1.5 (ca. 130 ml). Stir 10—15 minutes and separate the layers. Re-extract the aqueous layer with 100 ml MIBK at pH 1.0—1.5, 20°—25°C for 10—15 minutes, and separate. Combine the MIBK layers and add sodium ethyl hexoate in MIBK (a 2N solution) over 1.5 hours to pH 5.5 (ca. 315—320 ml). Stir for 0.5 hours at 20°—25°C and 0.5 hours at 0°—5°C, filter and wash with 75 ml MIBK and 100 ml methylene dichloride (MDC). Dry at 35°C, to give 125 g of monosodium 3-thienyl malonate.

Example 2

Preparation of 3-thienylmalonic acid from the monosodium salt

100 gm of monosodium 3-thienyl malonate is added to 200 ml water at a temperatue of 15°—20°C and is stirred until dissolved. Concentrated HCl (total 34 ml) is added over 15 minutes to pH 1.6—2.0. The solution is seeded with 3-thienylmalonic acid and stirred for 30 minutes during which time crystallization occurs. 40 gm of sodium chloride is added and the mixture is stirred for 15 minutes. Concentrated HCl (6 ml) is added over 15 minutes to pH 1.0—1.3. The mixture is cooled to 0°—5°C and stirred for 90 minutes at 0°—5°C. It is filtered, washed with 200 ml methylene dichloride and dried at 35°C overnight. This process gives the following average product.

| | | |
|---|---|---|
| Weight | : | 84—86g |
| Percent Acidity | : | 89—94% |
| Percent Yield | : | 96—98% |

**Claims**

1. A salt of 3-thienylmalonic acid of formula (II):

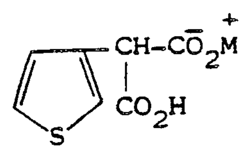

(II)

wherein $\overset{+}{M}$ represents an alkali metal ion or an ammonium ion.

2. The monosodium salt of 3-thienylmalonic acid.

3. A process for the preparation of 3-thienylmalonic acid which process comprises hydrolysing a 3-thienylcyanoacetate of formula (IV):

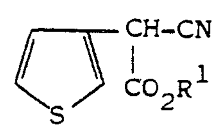

(IV)

wherein $R^1$ represents an alkyl or aryl group, with an alkali metal hydroxide, isolating a salt according to claim 1 and converting it to 3-thienylmalonic acid.

4. A process according to claim 3 wherein the monosodium salt of 3-thienylmalonic acid is isolated by precipitation.

5. A process according to claim 3 wherein $R^1$ is $C_{1-6}$ alkyl.

## Patentansprüche

1. Ein Salz der 3-Thienylmalonsäure der Formel (II):

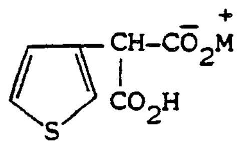

(II)

in welcher $\overset{+}{M}$ ein Alkalimetallion oder ein Ammoniumion darstellt.

2. Das Mononatriumsalz der 3-Thienylmalonsäure.

3. Ein Verfahren zur Herstellung von 3-Thienylmalonsäure, welches Verfahren das Hydrolysieren eines 3-Thienylcyanoacetats der Formel (IV):

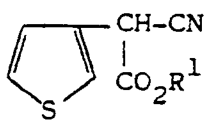

(IV)

in welcher $R^1$ eine Alkyl- oder Arylgruppe bedeutet, mit einem Alkalimetallhydroxid, das Isolieren eines Salzes nach Anspruch 1 und dessen Umwandlung in 3-Thienylmalonsäure umfaßt.

4. Ein Verfahren nach Anspruch 3, in welchem das Mononatriumsalz der 3-Thienylmalonsäure durch Ausfällen isoliert wird.

5. Ein Verfahren nach Anspruch 3, in welchem $R^1$ ein $C_{1-6}$-Alkyl darstellt.

## Revendications

1. Sel d'acide 3-thiénylmalonique de formule (II)

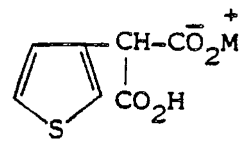

(II)

dans laquelle $\overset{+}{M}$ représente un ion métal alcalin ou un ion ammonium.

2. Sel monosodique de l'acide 3-thiénylmalonique.

3. Procédé de préparation de l'acide 3-thiénylmalonique caractérisé en ce qu'il comprend l'hydrolyse d'un 3-thiénylcyanoacétate de formule (IV):

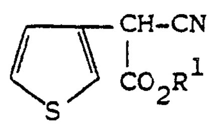

(IV)

dans laquelle $R^1$ représente un groupe alcoyle ou aryle, avec un hydroxyde de métal alcalin, l'isolation d'un sel suivant la revendication 1 et la conversion de celui-ci en acide 3-thiénylmalonique.

4. Procédé suivant la revendication 3, caractérisé en ce que le sel monosodique de l'acide 3-thiénylmalonique est isolé par précipitation.

5. Procédé suivant la revendication 3, caractérisé en ce que $R^1$ est un groupe alcoyle en $C_{1-6}$.